# EUROPEAN PATENT SPECIFICATION

(11) **EP 0 959 874 B1**
(45) Date of publication and mention of the grant of the patent: **02.06.2004**
(21) Application number: 97933287.1
(22) Date of filing: 02.07.1997
(51) Int. Cl.: A61K 9/12, A61K 9/127, A61K 9/00, A61K 31/58, A61K 38/13

(54) **HIGH DOSE LIPOSOMAL AEROSOL FORMULATIONS**
HOCHDOSIERTE LIPOSOMALE AEROSOLFORMULIERUNG
FORMULATIONS D'UN AEROSOL A LIPOSOMES FORTEMENT DOSE

(30) Priority: 03.07.1996 US 675654; 16.10.1996 US 731605
(43) Date of publication of application: 01.12.1999
(73) Proprietor: RESEARCH DEVELOPMENT FOUNDATION, Carson City, Nevada 89703 (US)
(72) Inventor: WALDREP, J., Clifford, The Woodlands, TX 77381 (US); KNIGHT, Vernon, Houston, TX 77027 (US); BLACK, Melanie B., The Woodlands, TX 77380 (US)
(74) Representative: McQueen, Andrew Peter
(86) International application number: PCT/US1997/011696
(87) International publication number: WO 1998/000111

(56) References cited:
- EP-A- 0 170 642
- EP-A- 0 260 241
- EP-A- 0 267 050
- WO-A-92/18105
- WO-A-93/15715
- WO-A-96/19199
- US-A- 4 906 476
- US-A- 5 049 388
- INT. J. PHARMACEUTICS, 15 August 1993, Volume 97, Abstract No. 02181677, WALDREP et al., "Cyclosporin A Liposomal Aerosol: Particle Size Calculated Respiratory Deposition".
- DISSERTATION ABSTRACTS INTERNATIONAL, 1995, Volume 57/02C, Abstract No. 01476461, FORNHEM C., "Allergen-Induced Late Airways Reaction in the Pig: Influence of Endogenous and Exogenous Glucocorticoids".
- WALDREP J.C. ET AL: 'Operating characteristics of 18 continuous-flow jet nebulizers with beclomethasone dipropionate liposome aerosol' CHEST vol. 105, no. 1, 1994, pages 106 - 110
- SCHWARZ L.A. ET AL: 'Delivery of DNA-cationic liposome complexes by small-particle aerosol' HUM.GENE.THER. vol. 7, no. 6, 1996, pages 731 - 741

## Description

The present invention relates generally to the fields of biochemical pharmacology and medicinal chemistry. More specifically, the present invention relates to high dose liposomal aerosol formulations of cyclosporin A.

### Description of the Related Art

In the lung, many different diseases have been treated successfully through utilization of aerosol delivery systems used to deposit drugs directly on to the pulmonary surfaces. For delivery in this manner, a variety of devices have been developed (for example, metered dose inhalers and dry powdered inhalers). Jet-nebulizers have been used clinically for aerosol delivery of water soluble drugs and micronized suspensions; however, their use with water insoluble, hydrophobic compounds has been limited.

The development of liposomal formulations compatible with aerosol delivery has allowed the jet nebulizer to deliver additional drugs. Utilization of liposomes for aerosol delivery has many advantages, including aqueous compatibility; sustained pulmonary release allowing maintenance therapeutic drug levels; and, further, liposomes facilitate intra-cellular delivery, particularly to alveolar macrophages.

The efficacy of localized, topical therapy via aerosols is determined by the amount of drug delivered to the sites of disease within the lung; and there are several different key parameters that determine the amount of delivery, thus, the therapeutic efficacy of aerosol formulations. For example, nebulizer design and variation, operating conditions (e.g., flow rate), and the presence of ancillary equipment (tubing, connectors, mouth pieces, face masks, and the like), are important variables. Thus, aerosol output efficiency can be increased through proper implementation of the proper nebulizer device. Inappropriate implementation of the device and/or imperfect parameters can affect inhaled dosages, delivery sites and influence the therapeutic outcome.

Drug formulation also is a critical factor regulating aerosol output efficiency and aerodynamic properties of drug-liposomes. It has been discovered that drug-liposome output efficiency can be increased through the utilization of liposomes formulated with low phase transition temperatures (see Waldrep et al., *J. of Aerosol Med.* 7:1994 (1994) and Waldrep et al., *Int'l J. of Pharmaceutics* 97:205-12 (1993)). An additional method to increase aerosol drug-liposome output is to increase the drug and phospholipid reservoir concentrations. Nebulization of some drug-liposome formulations at greater than 50 mg/ml results in clogging of the nebulizer jets; yet empty liposomal formulations up to 150 mg/ml have been successfully nebulized (see Thomas, et al., Chest 99:1268-70 (1991)). Further, the aerosol performance (output and particle size) is influenced in part by physicochemical properties such as viscosity and surface tension. Such variables affect the maximal drug-liposome concentrations compatible with aerosol delivery via the jet nebulizer.

Anti-inflammatory glucocorticoids have been used for the treatment of asthma and other severe inflammatory lung diseases for over forty years. More recently, aerosol glucocorticoid therapy has been used increasingly as a route of administration. Presently, there are several different, though structurally similar, topically active glucocorticoids-e.g., beclomethasone, budesonide, flunisolide, triamcinolone acetonide and dexamethasone--that are available in metered dose inhalers or dry powder inhalers for aerosol treatment of asthma and other inflammatory diseases of the lung. While systemic complications such as suppression of the hypothalamic-pituitary axis, cataract formation and growth inhibition are infrequent in asthmatics treated with inhaled glucocorticoids, localized side effects of candidiasis and dysphonia are more common, necessitating the use of accessory spacer devices. At present in the United States, there are no glucocorticoid formulations approved for nebulized administration, although micronized suspensions of beclomethasone and budesonide are employed in Europe and Canada.

US 5,049,388 teaches aqueous aerosol droplets containing a liposome interacted with a drug, e.g. cyclosporin A, delivered in a continuous phase of air or oxygen having a diameter less than 5 microns and a mass median aerodynamic diameter ranging from 1-3 microns. The reference discloses that a dose is regulated by, inter alia, the concentration of the liposome-drug preparation in the aerosol generator which ranges from 10-40 mg/ml of the liposome-drug preparation (this is the total concentration of both liposome and drug). US 5,049,388 makes no suggestion that a higher starting reservoir concentration can be effected for any of the liposome-drug combinations it discloses.

WO 96/19199 teaches a proliposomal composition having a drug, e.g., cyclosporin A, and a phospholipid, with a phase-transition temperature under 37°C so that it may be hydrated under physiological conditions, in a dry powder which is delivered to the respiratory tract via an inhaler. The proliposome is hydrated in the respiratory tract after inhalation via a dry powder inhaler or prior to delivery from a pressurized aerosol inhaler. The proliposomes are not nebulized and can aggregate within the aerosol in particles up to 1 mm.

EP-A-0267050 teaches a liposomal-drug aerosol which can be generated via nebulization to the liposome-drug nebulized aerosol described in WO 96/19199. However, EP-A-0267050 does not disclose cyclosporin A nor budesonide as drugs that can be incorporated into the liposome.

Waldrep et al (1993) Int. J. Pharmaceutics Vol. 97 Abstract No. 02181677 compares the properties of aerosols of beclomethasone dipropionate-DPLC liposomes generated by different commercially available jet nebulizers to determine if any or all of them generate aerosols with characteristics beneficial for liposome-drug delivery, e.g., mass median aerodynamic diameter of 1-3 microns. Waldrep et al teaches that aerosols of budesonide-lipid can be generated using the machines disclosed in the reference, however, the state of the art at the time of the reference limits the reservoir concentration and no suggestion is made to increase the reservoir concentration.

The present invention is drawn to concentrated, high dose cyclosporin-A-liposome aerosol formulations that provide maximal aerosol output with particle size ranges within the optimal range of 1-3 µm mass median aerodynamic diameter (MMAD). The prior art is deficient in such liposomal aerosol formulations. The present invention fulfills this long-standing need and desire in the art.

### SUMMARY OF THE INVENTION

The present invention provides a high dose cyclosporin A (CsA) liposome aerosol composition comprising up to about 30 mg/ml cyclosporin A in up to about 225 mg of a phospholipid/ml starting reservoir concentration.

In a preferred embodiment of the present invention, there is provided a high dose cyclosporin A liposome aerosol composition comprising up to about 20 mg/ml cyclosporin A in up to about 150 mg of dilauroylphosphatidylcholine(DLPC)/ml starting reservoir concentration.

In a most preferred embodiment of the present invention, there is provided a high dose cyclosporin A liposome aerosol composition comprising up to about 21.3 mg/ml cyclosporin A in up to about 160 mg of dilauroylphosphatidylcholine (DLPC) /ml starting reservoir concentration. Other phospholipids might be substituted for DLPC in the high dose the CSA-liposomal formulation.

Other and further aspects, features, and advantages of the present invention will be apparent from the following description of the presently preferred embodiments of the invention given for the purpose of disclosure. Is must be noted that any reference to budenoside liposomal preparations in the following pages is comparative and not part of the invention.

### BRIEF DESCRIPTION OF THE DRAWINGS

So that the matter in which the above-recited features, advantages and objects of the invention are attained and can be understood in detail, more particular descriptions of the invention summarized briefly above may be had by reference to certain embodiments which are illustrated in the appended drawings. These drawings form a part of the specification. It is to be noted, however, that the appended drawings illustrate preferred embodiments of the invention.

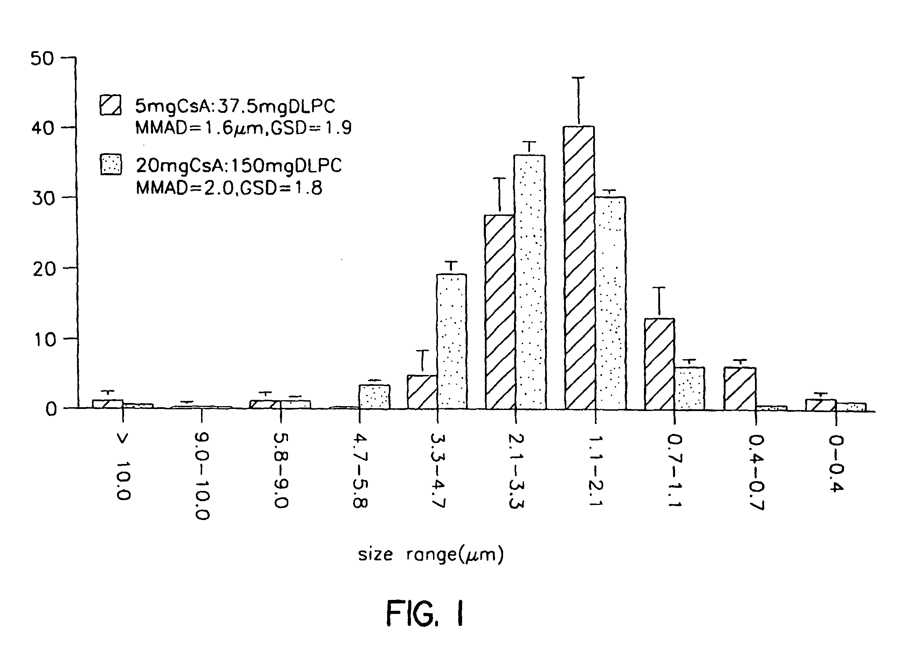

Figure 1 shows the aerosol distribution profile of high and low dose cyclosporin A-DLPC liposomal formulations nebulized with an Aerotech II nebulizer at a flow rate of 10 liters per minute as determined by the Andersen Cascade Impactor. The data (mean ± standard deviation) represent the fractional percentage of total cyclosporin A recovered on each stage of the impactor with associated size cut-off in µm (n=3 analyses). The mass median aerodynamic diameters (MMAD) and geometric standard deviations (GSD) were calculated on a log-probability plot.

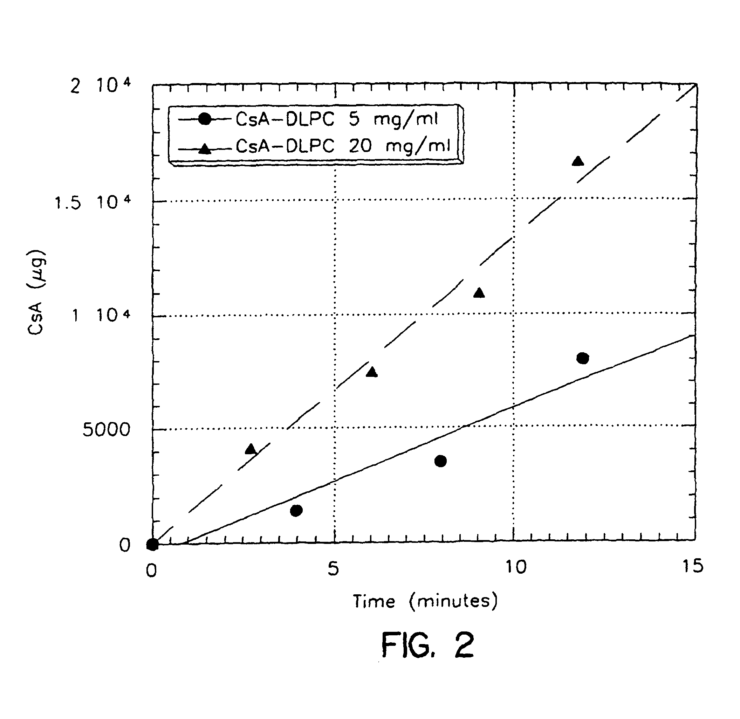

Figure 2 shows the cyclosporin A inhaled from high and low dose cyclosporin A-DLPC liposomal formulations nebulized with an Aerotech II nebulizer at a flow rate of 10 liters per minute as determined in a human lung simulation model. The values represent cyclosporin A collected at different nebulization times from aerosol samples by filters attached to a Harvard Respirator adjusted to a tidal volume (TV) of 500 ml and a rate of 15 breaths per minute (BPM).

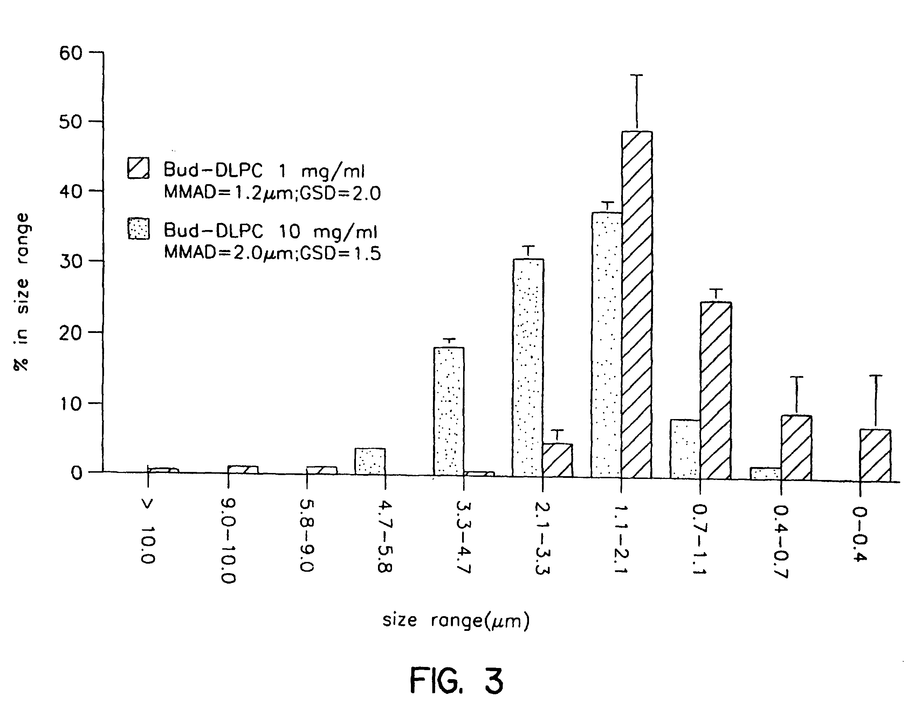

Figure 3 shows the aerosol distribution profile of high and low dose Budesonide-DLPC liposomal formulations nebulized with an Aerotech II nebulizer at a flow rate of 10 liters per minute as determined by the Andersen Casacade Impactor. The data (mean ± standard deviation) represent the fractional percentage of total cyclosporin A recovered on each stage of the impactor with associated size cut-off in µm (n=3 analyses). The mass median aerodynamic diameters (MMAD) and geometric standard deviations (GSD) were calculated on a log-probability plot.

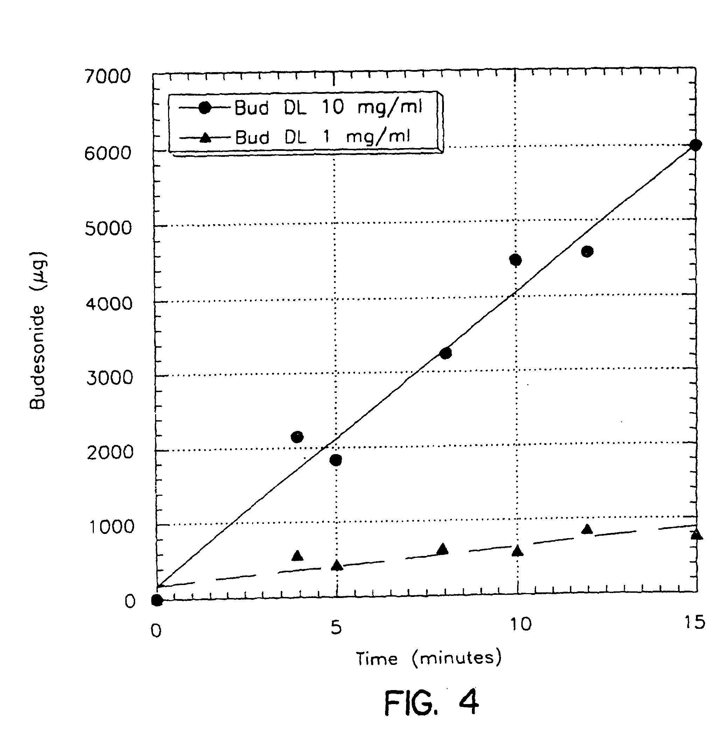

Figure 4 shows the Budesonide inhaled from high and low dose Budesonide-DLPC liposomal formulations nebulized with an Aerotech II nebulizer at a flow rate of 10 liters per minute as determined in a human lung simulation model with a tidal volume (TV) of 500 ml and 15 breaths per minute (BPM). The values represent Budesonide collected at different nebulization times from aerosol samples by filters attached to a Harvard Respirator adjusted to a tidal volume (TV) of 500 ml and a rate of 15 breaths per minute (BPM).

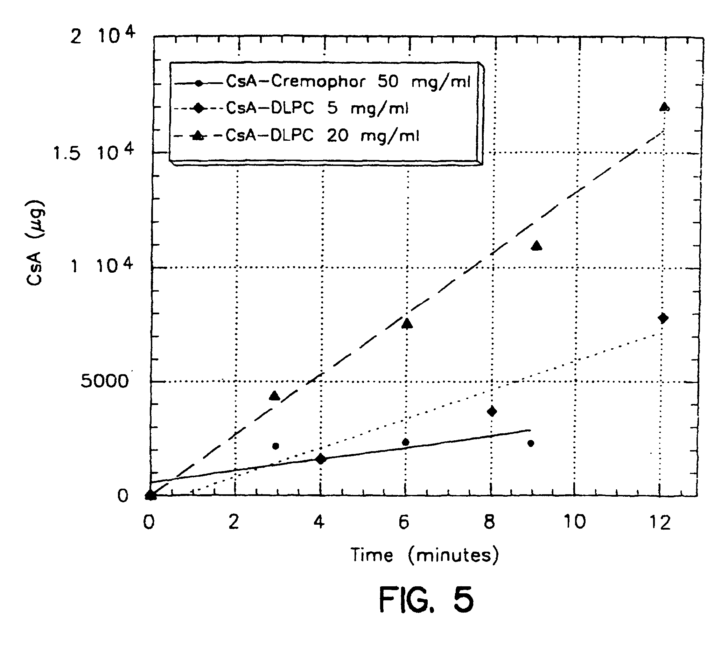

Figure 5 shows the time frame of simulated CsA concentrations inhaled from nebulized liposomal and cremophor formulations. Plotted are CsA-Cremophor (50 mg/ml; circles), CsA-DLPC (5 mg/ml; filled triangles), and CsA-DLPC (20 mg/ml; diamonds).

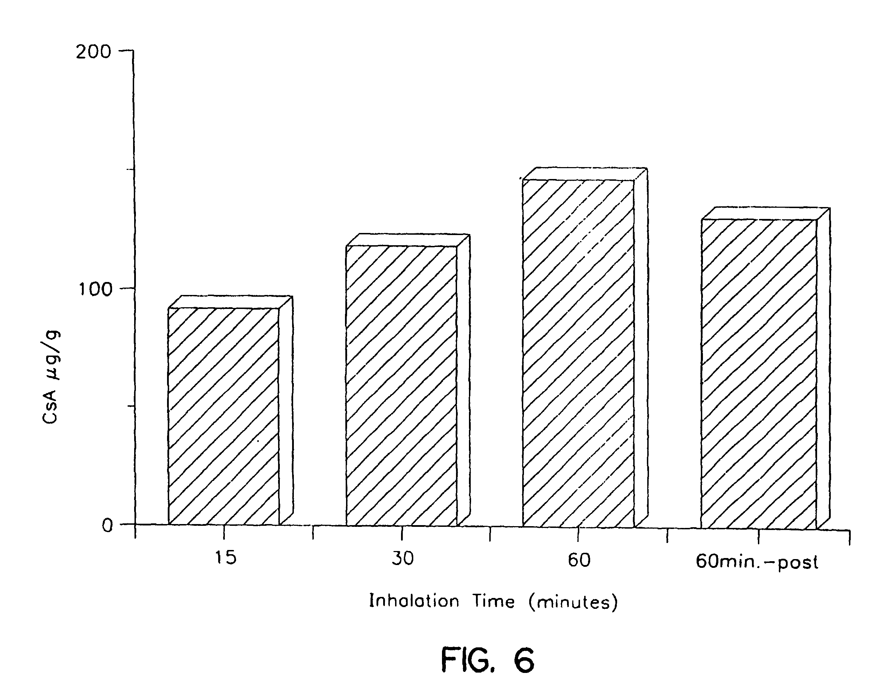

Figure 6 shows the concentration of pulmonary CsA, over an inhalation time in ICR mice (35g) after inhalation of nebulized high-dose CsA-DLPC (20 mg/ml).

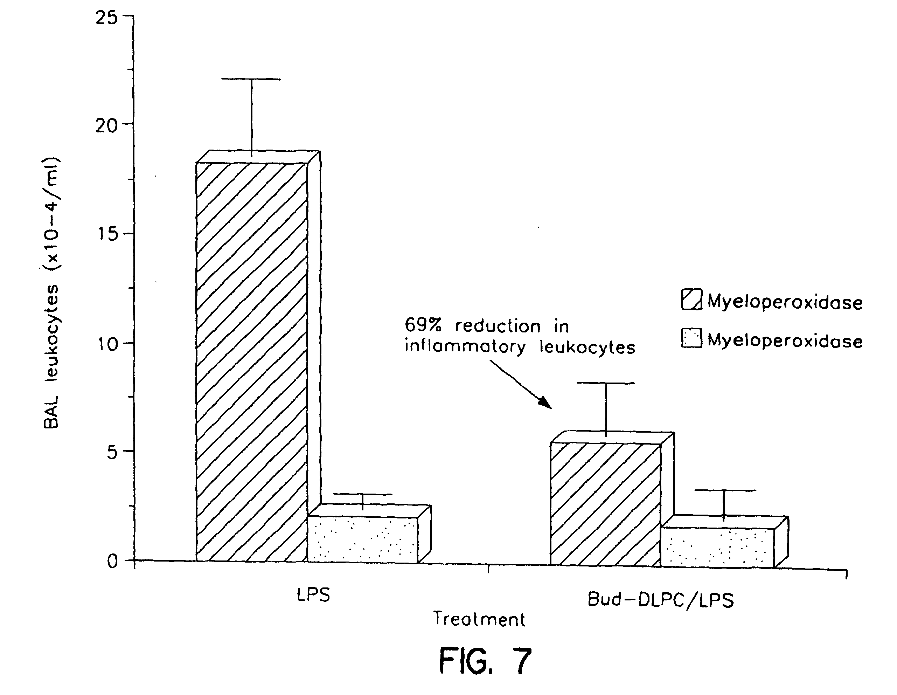

Figure 7 shows the anti-inflammatory effect of high dose Bud-DLPC on lung bronchiolaralveolar lavage (BAL) leukocytes in response to LPS (endotoxin) challenge.

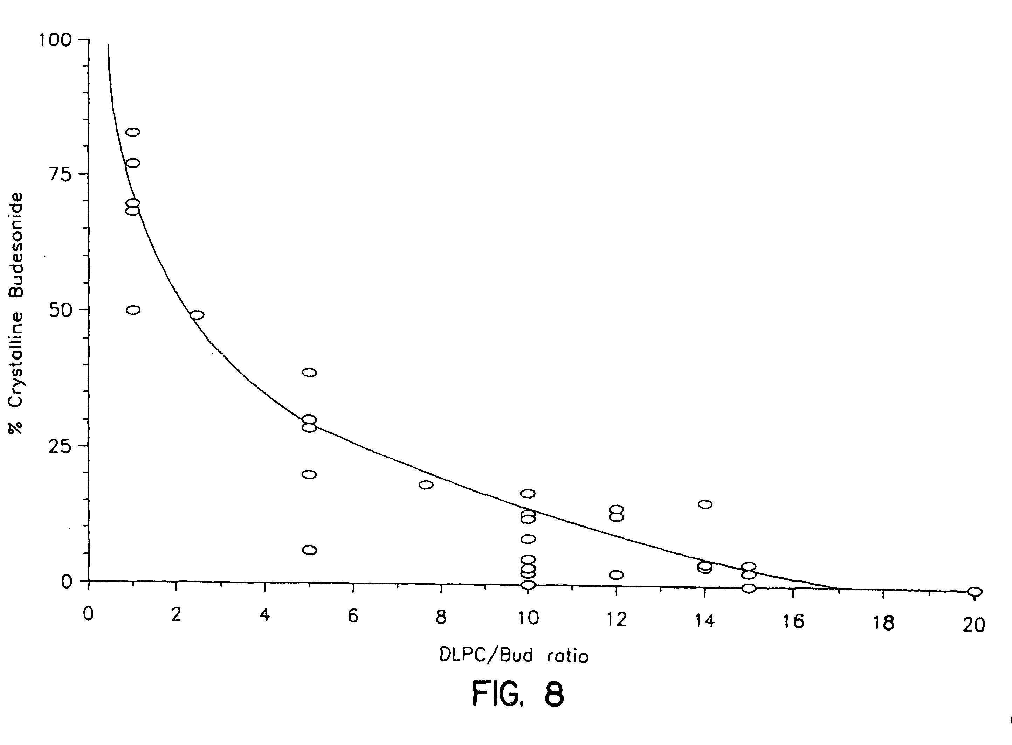

Figure 8 shows a percoll gradient analysis of Bud-DLPC liposomes.

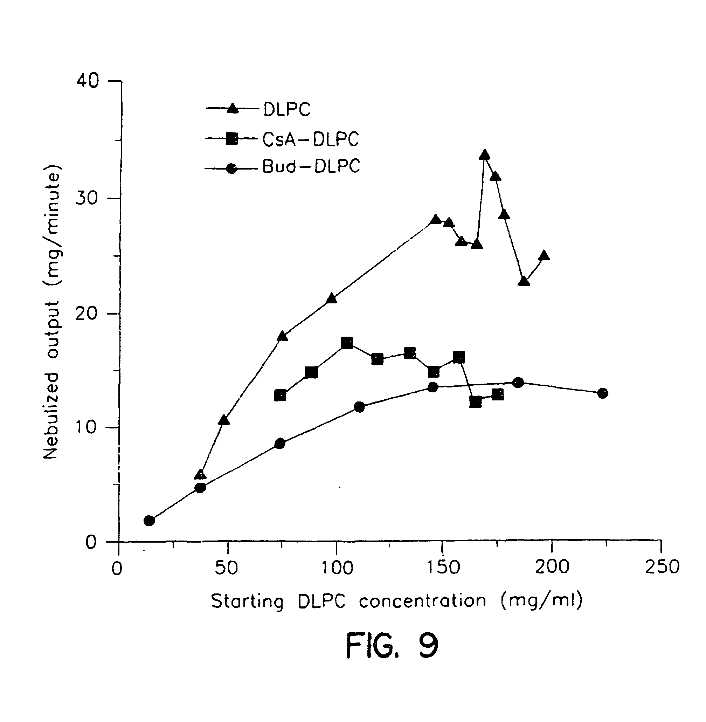

Figure 9 shows the aerosol DLPC output (mg/min.) of nebulized empty DLPC, CsA-DLPC, and Bud-DLPC liposomal formulations of increasing concentrations. Aerosols were generated with water tested and standardized Aerotech II nebulizers (initial starting volume of 5 mls; 10 L/min. flow rate) and paired samples were collected in AGI-4 impingers at 4-5 & 6-7 minutes of nebulization. DLPC concentrations were determined by HPLC analysis. Data presented is representative of the formulations tested at each indicated concentration and plotted by initial liposomal DLPC content (mg/ml).

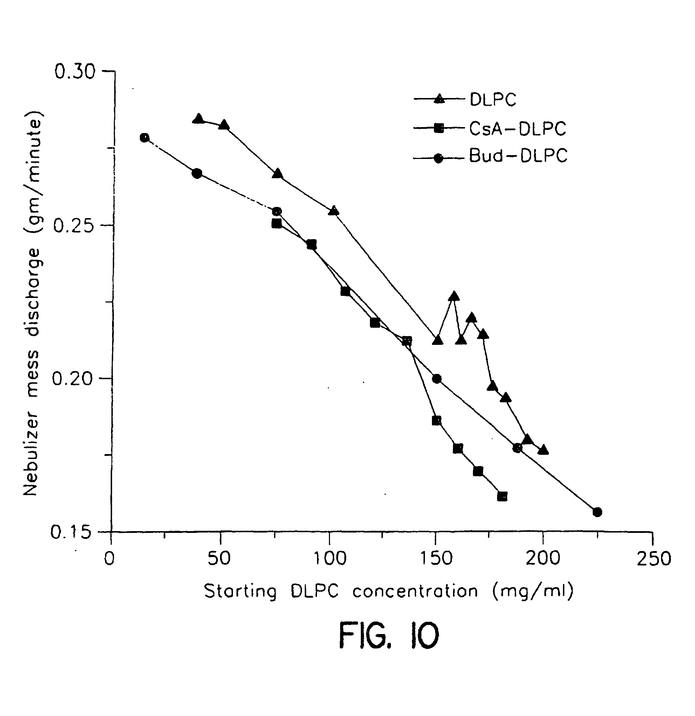

Figure 10 shows the mass discharge (gm/min.) of nebulized empty DLPC, CsA-DLPC, and Bud-DLPC liposomal formulations of increasing concentrations. Aerosols were generated with water tested and standardized Aerotech II nebulizers (initial starting volume of 5 mls; 10 L/min. flow rate) and the mass output determined using an analytical balance after 10 minutes of nebulization. Data presented is representative of the formulations tested at each indicated concentration and plotted by initial liposomal DLPC content (mg/ml).

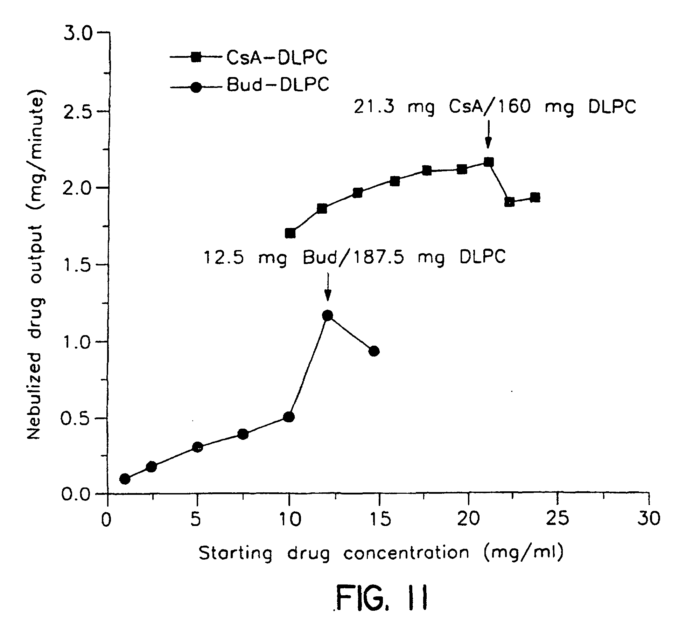

Figure 11 shows the aerosol CsA and Bud output (mg/min.) of nebulized CsA-DLPC and Bud-DLPC liposomal formulations of increasing concentrations. Aerosols were generated with water tested and standardized Aerotech II nebulizers (initial starting volume of 5 mls; 10 L/min. flow rate) and paired samples were collected in AGI-4 impingers at 4-5 & 6-7 minutes of nebulization. Drug concentrations were determined by HPLC analysis from aliquots of samples also analyzed for DLPC content (Fig. 1). Data presented is representative of the formulations tested at each indicated concentration and plotted by initial liposomal drug content (mg/ml).

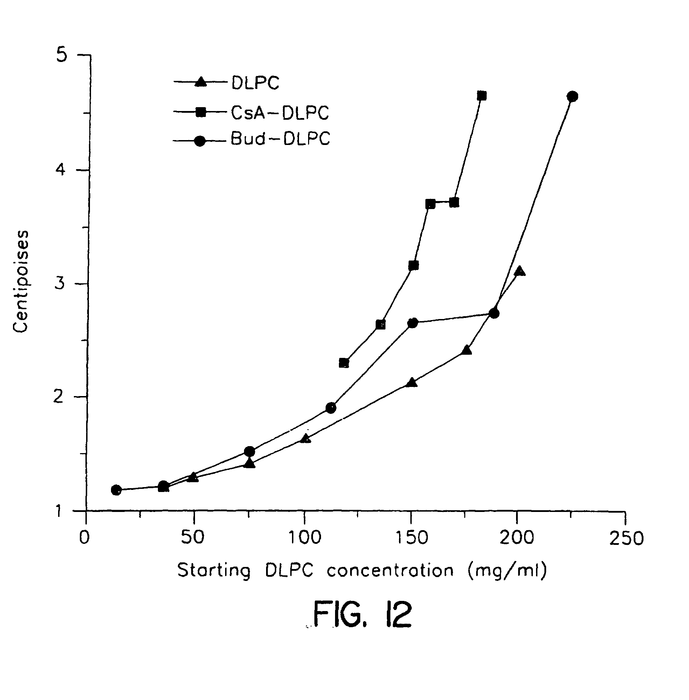

Figure 12 shows the viscosity (centipoises) analysis of empty DLPC, CsA-DLPC, and Bud-DLPC liposomal formulations of increasing concentrations (initial starting volume 10 mls; ambient room temperature). Data presented is the mean of 10 observations for each of the formulations tested at each indicated concentration and plotted by initial liposomal DLPC content (mg/ml).

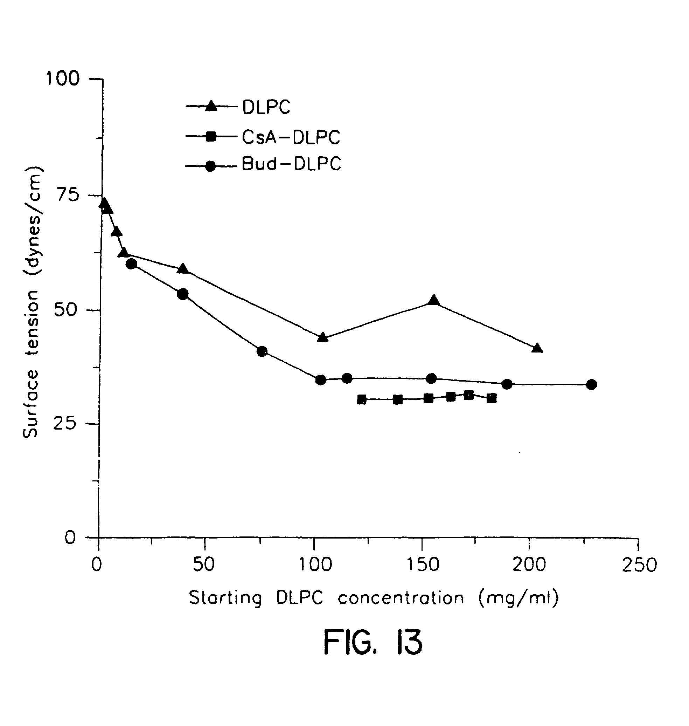

Figure 13 shows surface tension (dynes/cm) analysis of empty DLPC, CsA-DLPC, and Bud-DLPC liposomal formulations of increasing concentrations (initial starting volume 7 mls; ambient room temperature). Data presented is the mean of 10 observations for each of the formulations tested at each indicated concentration and plotted by initial liposomal DLPC content (mg/ml). Samples were also analyzed for viscosity.

### DETAILED DESCRIPTION OF THE INVENTION

One object of the present invention is to improve the efficiency of delivery of a high dose pharmaceutical compound-liposome aerosol composition. Hence, the present invention is drawn to the improved efficiency of delivery of a cyclosporin A-liposome aerosol. In a series of experiments, it was determined that the aerosol drug output could be improved through the utilization of liposomes formulated with low phase transition temperatures, such as DLPC (containing 12 carbon, saturated fatty acid side chains). It was also determined that certain nebulizers increase the aerosol drug-liposome output in the desired size range of 1-3 µm mass median aerodynamic diameter (MMAD). The cyclosporin A concentration employed in these early studies was 1.0 mg with 7.5 mg DLPC per ml of starting solution in the reservoir.

In 1993, the need to increase the cyclosporin A-liposome aerosol output by scaling up the formulation was recognized. This could be accomplished in different ways, such as selection of a more efficient nebulizer. The cyclosporin A-liposome aerosol output was scaled up through the implementation of the Aerotech II (ATII) nebulizer (from CIS-USA, Bedford, Mass). The ATII has approximately 50 percent increased aerosol output over the previously used Puritan Bennett 1600sj.

A second method of increasing aerosol drug-liposome output was to increase the reservoir concentration of drug and phospholipid in the liquid of the nebulizer reservoir. The cyclosporin A-DLPC liposome concentration of 5 mg cyclosporin A/37.5 mg per ml was successfully increased while achieving desired aerosol output in the range of 1-3 µm mass median aerodynamic diameter (MMAD). Using a human lung deposition model, analysis of this aerosol indicated that approximately 3.2 mg of cyclosporin A theoretically would deposit within the lung after a single 15 minute inhalation. Studies by the University of Pittsburgh group of lung allograft patients treated with aerosolized cyclosporin A (dissolved in ethanol or propyleneglycol) demonstrated clinical improvement (reversal of graft rejection) when 20 mg of cyclosporin A was delivered to the lung. Using the available cyclosporin A-DLPC liposome system requires approximately 2 hours of aerosol inhalation to deliver this amount. Such a prolonged, daily inhalation interval would likely be cumbersome for the patient, and requires 8 recharges of the nebulizer reservoir. Therefore, it was required to increase the cyclosporin A-DLPC reservoir concentration. However, it is well known in the prior art that it was not possible to nebulize liposomes greater than 50 mg/ml, since greater concentrations resulted in clogging of the nebulizer jets.

The present invention succeeded in achieving 20-30 mg/ml cyclosporin A:150-225 mg DLPC/ml starting reservoir concentration. The particle size was increased marginally by this change, shifting upward the MMAD of the aerosol to 2.0 µm from 1.6 µm as demonstrated with cyclosporin A-DLPC (5 mg/37.5mg) with no change in the GSD (Figure 1). The aerosol output of the "high-dose" 20-30 mg cyclosporin A-DLPC significantly was higher than the 5 mg cyclosporin A-DLPC.

As demonstrated in Figure 2 in a simulated human lung model, 15 minutes with high-dose cyclosporin A-DLPC, the required time period to deliver a putative therapeutic dose in lung allograft patients would be approximately 45 minutes or less. Certainly, this interval is based on dosing results by other investigators using other cyclosporin A aerosols. Since cyclosporin A-liposomes are theoretically more effective at a lower dose and less toxic than cyclosporin A in ethanol or propylene glycol, the inhalation interval would likely be much less. Increasing the cyclosporin A-DLPC higher than about 30 mg cyclosporin A -225 mg DLPC proved inefficient.

The present invention demonstrated the usefulness of a high dose cyclosporin A-DLPC liposome aerosol in the range of 20-25 mg cyclosporin A/150-200 mg DLPC per ml; although amounts up to 30 mg/ml would also constitute high dose. Other phospholipids might be substituted for DLPC in the high dose cyclosporin A-liposomal formulation. Representative examples of suitable phospholipids include egg yolk phosphatidylcholide, hydrogenated soybean phosphatidylcholide, dimyristoylphosphatidylcholide, dioleoyl dipalmitoylphosphatidylcholide and dipalmitoyl phosphatidylcholide.

High dose cyclosporin A-liposome aerosol proves to be useful in a variety of immunologically mediated lung diseases, such as, allograft rejection, bronchiolitis obliterans, allergy, hypersensitivities, and asthma, and proves useful in pediatric, adult, and elderly patients with different nebulizer systems. Different inhalation intervals would be required for treating the various disease entities.

Producing high dose cyclosporin A-DLPC liposomes are useful since high dose cyclosporin A aerosols are being used to treat lung transplant rejection episodes. In these studies, patients are being treated with nebulized cyclosporin A-cremophor (50 mg cyclosporin A/ml). As demonstrated in the Figure 5, the aerosol output of cyclosporin A-liposomes (5 mg/ml & 20 mg/ml) is markedly higher. The cyclosporin A-cremophor is very irritating but this aerosol has given some clinical benefit. Thus, the cyclosporin A-liposomes will be even better once tested in similar patients. The cyclosporin A-DLPC liposome aerosol are also effective in the treatment of asthma as described for oral cyclosporin A.

### EXAMPLE 1

### Liposome Formulation: Preparation of High Dose Drug/Liposomes

A lyophilization procedure has been developed in the present invention for optimal formulation of various drug/liposomes. It was ascertained that the optimal cyclosporin A to DLPC ratio was 1:7.5 by weight. To determine the maximum concentration compatible with nebulization, aerosol delivery formulations were produced at 10 to 30 mg cyclosporin A with 75 to 225 mg DLPC, at the optimal CsA to DLPC, ratio of 1:7.5 by weight. It was determined that formulations containing 21.3 mg cyclosporin A: 160 mg DLPC were optimal as judged by aerosol output and particle size for inhalation. For optimized high dose cyclosporin A-liposomes, 100 mg of cyclosporin A (Sandoz Pharmaceuticals or Chemwerth Chemical company) was mixed with 750 mg of synthetic alpha-lecithin: 1, 2-dilauroyl-sn-glycero-3-phosphocholine (DLPC from Avanti Polar Lipids). Working at 37°C in a warm room, the drug/DLPC was mixed in 20 ml of tertiary butanol with stirring as described in Waldrep et al., *Int,l J*. *of Pharmaceutics* 97:205-12 (1993). After mixing, the drug/lipid mixture was pipetted into glass vials, frozen rapidly, then lyophilized overnight to remove the t-butanol leaving a powder mixture. Multi-lamellar liposomes were produced by adding 10 ml of ultra pure water above the phase transition temperature (Tc) at 25°C to deliver a final standard drug concentration of 1-30 mg cyclosporin A: 7.5-225 mg DLPC per ml. The mixture was incubated for 30 minutes at room temperature with intermittent mixing to produce multilamellar vesicular liposomes. As an alternative, this liposomal formulation can be prepared by rotary evaporation. Aliquots were taken for determination of drug concentration by HPLC. This simple liposome preparation method was chosen since it can be scaled up easily for large batch preparation.

After swelling, the quality of the liposome preparations was checked for size and for the presence of drug crystals by microscopy using an eye piece, both before and after nebulization. The drug-lipid association (encapsulation efficiency) was determined using percoll gradient analysis as described in O'Riordan, et al., *J. of Aerosol Med.,* in press (1996). Size reduction of the multilamellar vesicular high dose cyclosporin A-DLPC drug-liposome formulations was not required prior to nebulization since the drug-liposomes (a heterogeneous starting mixture of 2.2 to 11.6 micrometers after swelling) are reduced further in size during nebulization (and continual reflux) by the shear forces generated by extrusion through the jet orifice of the nebulizer. The size range of these liposomes in aerosol droplets is 271-555 nm. Aqueous particles in aerosol contain one to several liposomes. The diameter of the liposomes is smaller than the aqueous aerosol particles in which they are carried (see Waldrep et al., *Int'l J. of Pharmaceutics* 97:205-12 (1993)). After swelling, the formulations are used for nebulization within several hours. Sterile formulations can be kept months at room temperature or in the refrigerator.

For formulation of high dose budesonide-DLPC liposomes, the optimal drug to lipid ratio was determined by testing different formulations at Budesonide-DLPC ratios of 1:1 to 1:20. The ratio of 1:15 (by weight) was selected as optimal for the high dose Budesonide-DLPC formulation. The high dose formulation is produced by mixing 10-150 mg of Budesonide with 150-2250 mg of DLPC (as described above for cyclosporin A-DLPC). Working at 37°C in a warm room, the drug/DLPC was mixed in 20 ml of tertiary butanol with stirring. After mixing, the drug/lipid mixture was pipetted into glass vials, frozen rapidly, then lyophilized overnight to remove the t-butanol, leaving a powder mixture. Multi-lamellar liposomes were produced by adding 10 ml of ultra pure water above the phase transition temperature (Tc) at 25°C to deliver a final standard drug concentration of 1-15 mg Budesonide: 15-225 mg DLPC per ml of solution. The mixture was incubated for 30 minutes at room temperature with intermittent mixing to produce multilamellar vesicular liposomes. Aliquots were taken for determination of drug concentration by HPLC. As an alternative, this liposomal formulation can be prepared by rotary evaporation. Figure 8 shows a percoll gradient analysis of Bud-DLPC liposomes (see O'Riordan, et al., *J. of Aerosol Med.,* in press (1996)). Once swollen, the multilamellar vesicular Budesonide-DLPC liposomes were stable for several weeks at room temperature. Sterile preparations were stable for months. Benzalkonium chloride (10 mg/liter) can be added as a preservative.

### EXAMPLE 2

### Liposome Aerosols: Aerosol Drug-Liposome Treatment

For the generation of drug-liposome aerosols, the Aerotech II nebulizer (CIS-USA, Bedford USA) was used although other commercial nebulizers could be employed. The ATII is a high-output, efficient nebulizer demonstrated to produce liposome aerosols in the optimal size range of 1-3 µM MMAD for peripheral lung delivery (see Vidgren, et al., *Int'l J. of Pharmaceutics* 115:209-16 (1994). A source of dry air was delivered to the nebulizer and its internal drying air intake via a regulated flow meter was at 10 liters/minute. An initial reservoir volume of 5 ml is sufficient for 15-20 minutes of aerosol. Longer treatment intervals would require refilling the reservoir.

### EXAMPLE 3

### Drug-liposome aerosol particle size distribution

Aerodynamic particle sizing of the drug-liposome aerosols was determined as described in Waldrep et al., J. of Aerosol Med. 7:1994 (1994), using an Andersen 1 ACFM non-viable ambient particle sizing sampler (Graseby Andersen Instruments Inc., Atlanta, GA) as a simulator of the human lung (Andersen). Drug-liposome aerosols generated from the ATII nebulizer were collected using a vacuum pump (1 ACFM) by impaction on 8 aluminum stages at a standard sampling interval of 0.5 minutes for each experiment. Drug concentrations in aerosol droplets between 0-10 µm sizes were collected on each stage (0=9.0-10.0 µm; 1=5.8-9.0 µm; 2=4.7-5.8 µm; 3=3.3-4.7 µm; 4=2.1-3.3 µm; 5= 1.1-2.1 µm; 6= 0.65-1.1 µm; 7= 0.43-0.65 µm) and determined after elution with 10 ml of ethanol or methanol and HPLC analysis. An USP artificial throat attached to the inlet port of the impactor is used to remove few aerosol particles larger than 10 µm. The final stage used a glass fiber collection filter. After determination of the drug concentrations for each stage by HPLC, the mass median aerodynamic diameter (MMAD) and geometric standard deviation (GSD) of the drug-liposomes was calculated on a log probability scale with the effective cutoff diameter as the ordinate and the cumulative percent less than the size range (by concentration) as the abscissa (KaleidaGraph 3.0). The MMAD and GSD are determined by the liposomal drug content distributed within the array of droplets comprising the aerosol (see Waldrep et al., *Int'l J. of Pharmaceutics* 97:205-12 (1993)). The droplet array rather than the liposome size determined the MMAD and the GSD. The validity of this method for calculation of MMAD & GSD was verified independently using a Model 3300 TSI Laser Aerosol Particle Sizer.

### EXAMPLE 4

### Estimation of Inhaled Dosage

For the determination of the estimated inhaled dosage of Bec-DLPC liposome, nebulized samples were collected in a simulated human lung system as described by Smaldone et al., Am *Rev Respir Dis* 143:727-37 (1991). Using a Harvard Respirator, aerosol samples were collected onto Whatman GF/F filters from the ATII nebulizer (10 L/minute flow rate) using 15 breaths per minute with tidal volumes of 500 ml. This basal tidal volume of 500 for males (450 for females) was determined from a nomogram adjusted for breathing frequency, weight, and sex. Aerosol samples were collected over a fifteen minute nebulization interval. The amount of cyclosporin A or Budesonide deposited on the filters was determined after extraction by HPLC analysis.

### EXAMPLE 5

### Analysis of Pulmonary Cyclosporin A: solid Phase Extraction

The following steps were performed:
1. Mouse lung tissue, after inhalation of cyclosporin A-DLPC liposome aerosol, was obtained. An internal CSD standard of 10 µg (10 µl of a 1 mg/ml stock) was added. The tissue was homogenized either in a blender or Wig-L-Bug tubes (using 4-5 beads per tube).
2. The homogenized tissue was extracted in 1 ml ultra pure water for 1-2 minutes. These volumes are for one tissue sample and are diluted if more than one tissue sample is pooled.
3. 2 ml of 98 percent Acetonitrile/ 2 percent Methanol was added and the sample was vortexed.
4. The sample was centrifuged at full speed 20 minutes; and the supernatant was transferred to a clean tube and centrifuged 10 minutes on full speed.
5. The supernatant was collected and 5 ml ultra pure water for every 1 ml used in tissue extraction was added.
6. A Sep-Pak C18 column (Waters Sep-Pak Light for single mouse tissue) was prepared and was washed with 5 ml 95 percent Ethanol and 5 ml ultra pure water. The sample was added slowly and washed with 5 ml ultra pure water, and 5 ml 50 percent Acetonitrile.
7. The eluate was transferred to a collection tube and eluted with 1 ml Methanol followed by 0.5 ml water.
8. Contaminants were removed by washing the eluate twice with 1.5 ml hexane and discarding the top layer.
9. The extracted eluate was evaporated to dryness using the reacti-vap temperature set on low with minimal air flow.
10. Reconstitution was in 0.3 ml CSA mobile phase and sample on HPLC.

### EXAMPLE 6

### Drug Analysis By High Pressure Liquid Chromatography (HPLC): Budesonide analysis

The HPLC assay was utilized for multiple purposes to determine: the Budesonide content of liposome formulations, the encapsulation efficiency, and the Budesonide content of aerosol samples obtained with the lung simulator. Budesonide concentrations were determined by HPLC analysis using a Waters WISP 717 autosampler and a Waters Nova-Pak C18 (3.9x150 mm) column at room temperature. Peak detection was performed at 238 nanometers using a variable UV/Vis wavelength detector with quantification by a Waters Millenium 2010 Chromatography Manager Version 2.15. The mobile phase utilized for these studies was 50:50 ethanol/water at a flow rate of 0.6 ml per minute (see Andersson & Ryrfeldt, *J Pharm Pharmacol* 36:763-65 (1984)). Samples for analysis were dissolved directly into ethanol (to solubilize the liposomes). Drug standards were prepared from ethanol stocks kept at -80°C.

### EXAMPLE 7

### Drug Analysis By High Pressure Liquid Chlomatography (HPLC):Cyclosporin A Analysis

The cyclosporin A in liposomal formulations (to determine cyclosporin A content and encapsulation efficiency) and in aerosol samples was determined by HPLC. A Waters (Milford, MA) WISP automatic sample injector and a Supelco LC-1 column heated to 75°C was used in the assay. The mobile phase was 50 percent acetonitrile, 20 percent methanol and 30 percent water (see Charles et al., *Ther. Drug Monitor.* 10:97-100 (1988)). Peaks were detected at 214 nm using a variable wavelength detector and quantified with the Waters Millenium 2010 Chromatography Manager version 2.15. Samples for analysis were dissolved directly in methanol (to solubilize the liposomes). Drug standards were prepared from methanol stocks kept at -80°C.

### EXAMPLE 8

### Drug Analysis By High Pressure Liquid Chromatography (HPLC): DLPC Analysis

A modification of the HPLC protocol of Grit and Commelin, *Chem. & Phys. of Lipids* 62:113-22 (1992), was used. A Waters 717 WISP automatic sample injector and a Sperisorb S5 amino column (,25 cm x 4.6 mm, 5µm) was utilized with acetonitrile, methanol, and 10 mM ammonium/trifluoroacetic acid, pH 4.8 (64:28:8 v:v:v) mobile phase. Peaks were detected with a mass evaporative detector (SEDEX 55, Sedre, France) and quantified with the Waters Millenium 2010 Chromatography Manager Version 2.15. Samples for analysis were dissolved directly in ethanol or methanol (to solubilize the liposomes).

### EXAMPLE 9

### Lung Models For Drug Testing In Mice: Acute Inflammation Assay: (LPS) Bronchiolar Lavage Technique

Gram negative cell wall lipopolysaccharide (LPS) was used for the reproducible induction of acute pulmonary inflammation in mice. A 10 minute exposure interval to *E. coli* 055:B5 LPS (Sigma) aerosol generated from the PBsj 1600 nebulizer (100 µg/ml reservoir concentration; 60 ng delivered dose) induced a strong phlogistic response as determined by the accumulation of PMN in the alveoli in response to the elaboration of chemotactic cytokines (detectable at 3 hours; peak response at 6 hours post stimulus). At varied times after exposure to LPS aerosol, the mice were sacrificed by methoxyflurane anesthesia and exsanguinated via the abdominal aorta. The trachea was surgically exposed and cannulated with PE50 tubing (outer diameter 0.965 mm, Clay Adams). Using a total volume of 2.0 ml Hank's Balanced Salt Solution (HBSS; Ca/Mg free with EDTA), the lungs are lavaged 5 times with approximately 1.0 ml volume. The yield was typically 85 percent recovery of lavage fluid. The resulting white cells were counted on a hemocytometer, cytocentrifuged and stained. From the differential count, drug effects were noted by a decrease in the white cell count and by reduced numbers of PMN and/or myeloperoxidase positive cells relative to the resident macrophages and/or myeloperoxidase negative cells. This assay was used as the standard to test drug-liposome aerosol regimen for biological activity through diminution of the acute inflammatory cellular influx of the airways. Figure 7 shows the anti-inflammatory effect of high dose Bud-DLPC on lung bronchiolaralveolar lavage (BAL) leukocytes in response to LPS (endotoxin) challenge.

### EXAMPLE 10

### Cytology: Lung Lavage

The cell preps (lavage, thymocytes, lymph node, or splenocytes) were counted on a hemocytometer, cytocentrifuged onto slides (using the Miles Cyto-Tek), and stained with Wright-Giemsa. May-Grunwald-Giemsa, or leukocyte peroxidase stain depending on the preparation. The differential count was determined by microscopic observation under oil immersion. Biological effects of the drug-liposome aerosol regimen were noted by a decrease in the total white cell count and by reduced numbers of PMN or myeloperoxidase positive cells relative to the resident macrophages.

### EXAMPLE 11

### Inhibition of Antigen/Mitogen Induced Lymphocyte Blastogenesis In Vitro by CsA Isolated from Mouse Lung Tissues after Aerosol Delivery of CsA-DLPC Liposomes

**Table 1**

| ANTIGEN/MITOGEN | AVERAGE CPM | % INHIBITION |
|---|---|---|
| Media | 2,171 | |
| OVA | 13,640 | |
| OVA + CsA 1 µg/ml | 2,173 | 99.9 |
| Media | 517 | |
| ConA | 24,341 | |
| ConA+ CsA 1 µg/ml | 3,041 | 89.4 |
| OVA = ovalbumin 250 µg/ml; ConA = concanavlin A 1 µg/ml; CPM = ³HTdR counts per minute average of 3. | | |

### Biological Activity of CsA Delivered to the Lung by Liposome Aerosol

For testing, a primary immune response was generated within the bronchiolar-associated lymphoid tissues and lung-associated lymph nodes within the mediastinum. After local intranasal immunization of Balb/c mice with alum precipitated ovalbumin (AP-OVA (80 µg) supplemented with Bordetella pertussis vaccine), the mice are sacrificed 7 days later, the mediastinum are removed and lymphocytes isolated for in vitro analysis. The proliferation assay consists of alterations in the stimulation of lymphocytes after activation with the sensitizing antigen ovalbumin or with the non-specific T-cell mitogen, Con A plus co-culture with CsA isolated from mouse lung tissues by solid phase extraction and quantified by HPLC. The uptake of ³[H]-TdR into DNA was determined at 48-72 hours. Inhibition of specific or non-specific lymphocyte activation was demonstrated by an abolition or reduction in the antigen-specific stimulation or in the inhibition of mitogen responsiveness.

### EXAMPLE 12

### Physicochemical Analysis:

Surface tension & viscosity: The surface tension (dynes/cm) was measured using a Tensiomat (Model 21, Fisher Scientific, Indiana, PA). A platinum iridium ring of known dimension was raised from the surface of the liquid to be tested under precisely controlled conditions. "Apparent" values read from the instrument were multiplied by a correction factor, F, incorporating dimensions of the measuring ring, density of the liquid, and other variables (according to the manufacturer's instructions). Viscosity measurements were performed using a Gilmont Falling Ball Viscometer (Gilmont Instruments, Barrington, IL). The viscosity in centipoises was determined at ambient room temperature.

Size measurements of drug-liposomes: The particle size of drug liposomal solutions was measured by quasielastic light scattering with a Nicomp Model 370, Submicron Particle Sizer, (Program Version 5.0, Nicomp Particle Sizing Systems, Santa Barbara, CA). Drug-liposome samples dispersed in water were analyzed according to the manufacturer's instructions and the data was expressed as intensity weighted vesicle size. Drug-liposome mean particle diameters were measured from reservoir samples initially, after 10 minutes of nebulization, and on aerosol samples recovered using the AGI-4 impinger as described by Waldrep et al., *Int'l J. of Pharmaceutics* 97:205-12 (1993).

The results in Figure 9 (plotted by DLPC concentration) demonstrate that there is increased aerosol output of DLPC liposomes up to 170 mg/ml, with reduction in output at higher concentrations. Extension of these data to CsA-DLPC liposomes yielded similar results, with maximal liposome aerosol output with 21.3 mg CsA: 160 mg DLPC/ml (Figure 9). For Bud-DLPC liposomes, the maximum aerosol DLPC output was demonstrated with the formulation consisting of 12.5 mg Bud:187.5 mg DLPC/ml. Analysis of nebulizer liquid vehicle discharge demonstrated in Figure 10 (plotted by DLPC) shows concentration dependent, reduced output as determined by the mass aerosolized/minute.

With increasing liposome concentrations, there are similar concomitant increases in aerosol output up to a critical point (Figure 11) (plotted by drug concentration). Measurements of aerosolized drug outputs of CsA and Bud by HPLC analysis demonstrated maximal concentrations for nebulization (Figure 11). For CsA-DLPC liposomes the maximum output was at 21.3 mg CsA: 160mg/ml. For Bud-DLPC, the maximum was at 12.5 mg Bud: 187.5 mg DLPC. Physicochemical analyses of these liposome formulations demonstrated parallel increases in viscosity (plotted by DLPC concentration) (Figure 12). The results for DLPC, Bud-DLPC and CsA-DLPC were similar. The viscosities of Bud-DLPC formulations were about 20 percent less than empty DLPC alone. Viscosities of CsA-DLPC were consistently the lowest and unchanging between 16 mg CsA/120 mg DLPC and 24 mg CsA/180 mg DLPC/ml. These results suggest that for each formulation there is a maximum viscosity compatible with nebulized aerosol output; above this threshold there is no added output with increased drug-liposome concentrations.

The results in Figure 13 (plotted by DLPC concentration) demonstrate that the addition of CsA and Bud to DLPC liposomes causes a reduction in the formulation surface tension. The reduction in surface tension was, to a point, concentration dependent, reaching a plateau around 100 mg DLPC/ml. There was no clear association between aerosol output of the liposomal formulations and surface tension. However, with increasing concentration of liposome formulations, there was an inverse relationship noted between surface tension and viscosity measurements.

Analysis of drug-liposome formulations prior to nebulization by quasielastic light scattering demonstrated a heterogeneous starting size range of approximately 2.2 to 11.6 µm (this is at or near the upper accuracy limit of Nicomp 370). After nebulization, there were minimal differences detected among any of the formulations. The size range of liposomes within the nebulizer reservoir were 294 to 502 nm and aerosol samples collected by the AGI-4 impinger ranged from 271 to 555 nm.

High dose formulations of drug-liposomes consisting of 10 mg Bud: 150 mg DLPC and CsA 20 mg: DLPC 150 mg were selected for further aerosol studies. Analysis with the Andersen Cascade Impactor demonstrated values of 2.0 µm MMAD/1.5 GSD for Bud-DLPC and 2.0µm/1.8 for CsA-DLPC (Table 2). Analysis of these formulations in a simulated human lung model at 15 BPM and 500 ml tidal volume demonstrates that a 3 minute inhalation interval would be required to inhale a 1,000 µg daily dose of Bud in liposomes, up to 5,000 µg could be inhaled in 12 minutes (Table 2). The results of CsA-DLPC inhalation in the simulated lung model demonstrated that with high dose CsA-DLPC, 4 minutes would be required to inhale 5000µg nebulized CsA in liposomes; 11.5 minutes would be required to inhale 15,000 µg of CsA (Table 2). These results demonstrate the high capacity of liposomes for aerosol drug delivery.

**Table 2**

| Aerosol Analysis and Inhaled Concentrations of Nebulized High Dose Bud-DLPC and CsA-DLPC Liposomal Formulations | | |
|---|---|---|
| Bud 10 mg-DLPC 150 mg | 1.000µg Dose | 5.000µg Dose |
| 2.0 µm MMAD* | 3 minutes | 12 minutes |
| 1.5 GSD | inhalation # | inhalation # |
| | | |

| CsA 20 mg-DLPC 150 mg | 5.000µg Dose | 15.000µg Dose |
|---|---|---|
| 2.0 µm MMAD* | 4 minutes | 11.5 minutes |
| 1.8 GSD | inhalation # | inhalation # |

| | | |
|---|---|---|
| * Andersen Cascade Impactor (mean of 3 determinations). # Human lung simulation Model (15BPM/500 ml TV) dosage calculated by linear regression analysis (Bud-DLPC n = 3; CsA-DLPC n = 2 analyses). | | |

The present invention is directed to a high dose cyclosporin A liposome aerosol composition comprising up to about 30 mg/ml cyclosporin A in up to about 225 mg of a phospholipid/ml starting reservoir concentration. Preferably, the liposome aerosol composition comprises up to about 21.3 mg/ml cyclosporin A in up to about 160 mg of a phospholipid/ml starting reservoir concentration. Generally, in the cyclosporin A liposome aerosol composition of the present invention, the particle size as measured by the mass median aerodynamic diameter is from about 1.0 µm to about 3.0 µm. Further, in the cyclosporin A liposome aerosol composition, the ratio of cyclosporin A to phospholipid is from about 1 to about 7.5. Preferably, the phospholipid is selected from the group consisting of egg yolk phosphatidylcholide, hydrogenated soybean phosphatidylcholide, dimyristoyphosphatidylcholide, diolyeolyldipalmitoyleolyl phosphatidylcholide and dipalmitoyl phosphatidylcholide. In general, the cyclosporin A liposome aerosol composition of the present invention may be used to treat an immunologically mediated lung disease. Preferably, such an immunologically mediated lung disease is selected from the group consisting of allograft rejection, bronchiolitis obliterans, allergy, hypersensitivities and asthma.

An individual having an immunologically mediated lung disease may be treated by a method comprising the step of administering to said individual a pharmacologically acceptable dose of a high dose cyclosporin A liposome aerosol composition. Preparation of suitable pharmaceutical compositions and concentrations for administration will be readily apparent to those having ordinary skill in this art given the teachings herein.

The present invention is also directed to a high dose cyclosporin A liposome aerosol composition comprising up to about 30 mg/ml cyclosporin A in up to about 225 mg of dilauroylphosphatidylcholine/ml starting reservoir concentration.

## Claims

1. A high dose cyclosporin A liposome aerosol composition comprising 5 mg/ml to 30 mg/ml cyclosporin A and 37.5 mg to 225 mg of a phospholipid/ml starting reservoir concentration wherein a mass median aerodynamic diameter of a particle of said liposome comprising. the aerosol is 1.0µm to 3.0µm.

2. The cyclosporin A liposome aerosol composition of claim 1, comprising 5 mg/ml to 21.3 mg/ml cyclosporin A and 37.5 mg to 160 mg of a phospholipid/ml starting reservoir concentration.

3. The cyclosporin A liposome aerosol composition of claim 2, wherein the phospholipid is dilauroylphosphatidylcholine.

4. The cyclosporin A liposome aerosol composition of claim 1, wherein the ratio of cyclosporin A to phospholipid is from 1 to 7.5.

5. The cyclosporin A liposome aerosol composition of claim 1, wherein the phospholipid is selected from the group consisting of egg yolk phosphatidylcholine, hydrogenated soybean phosphatidylcholine, dimyristoylphosphatidylcholine, dilauroylphosphatidylcholine, dioleoydipalmitoylphosphatidylcholine and dipalmitoylphosphatidylcholine.

6. The cyclosporin A liposome aerosol composition of claim 1, for use in the treatment of an immunologically mediated lung disease.

7. The cyclosporin A liposome aerosol composition of claim 6, wherein said immunologically mediated lung disease is selected from the group consisting of allograft rejection, bronchiolitis obiterans, allergy, hypersensitivities and asthma.

## Patentansprüche

1. Hochdosierte Aerosolzusammensetzung aus Cyclosporin A-Liposomen, die folgendes aufweist:
5 bis 30 mg/ml Cyclosporin A und 37,5 bis 225 mg eines Phospholipids/ml einer zugrundeliegenden Vorratskonzentration, wobei der massegemittelte aerodynamische Durchmesser eines Partikels des Liposoms, welches das Aerosol umfaßt,
1,0 µm bis 3,0 µm beträgt.

2. Aerosolzusammensetzung aus Cyclosporin A-Liposomen nach Anspruch 1,
die folgendes aufweist:
5 bis 21,3 mg/ml Cyclosporin A und 37,5 bis 160 mg eine Phospholipids/ml einer zugrundeliegenden Vorratskonzentration.

3. Aerosolzusammensetzung aus Cyclosporin A-Liposomen nach Anspruch 2,
wobei das Phospolipid Dilauroylphosphatidyl-Cholin ist.

4. Aerosolzusammensetzung aus Cyclosporin A-Liposomen nach Anspruch 1,
wobei das Verhältnis zwischen Cylosporin A und Phospholipid 1 zu 7,5 beträgt.

5. Aerosolzusammensetzung aus Cyclosporin A-Liposomen nach Anspruch 1,
wobei das Phospholipid aus der Gruppe ausgewählt ist, die aus Phosphatidyl-Cholin von Eigelb, Phosphatidyl-Cholin von hydrierten Sojabohnen, Dimyristoylphosphatidyl-Cholin, Dilauroylphosphatidyl-Cholin, Dioleoyldipalmitoylphosphatidyl-Cholin und Dipalmitoylphosphatidyl-Cholin.

6. Aerosolzusammensetzung aus Cyclosporin A-Liposomen nach Anspruch 1 zur Verwendung bei der Behandlung einer immunologisch vermittelten Lungenkrankheit.

7. Aerosolzusammensetzung aus Cyclosporin A-Liposomen nach Anspruch 6,
wobei die immunologisch vermittelte Lungenkrankheit aus der Gruppe ausgewählt ist, die aus einer Alutransplantat-Abstoßung, Bronchiolitis obiterans, Allergie, Hyperempfindlichkeiten und Asthma besteht.

## Revendications

1. Composition de liposomes à forte dose de cyclosporine A en aérosol comprenant une concentration de départ dans le réservoir de 5 mg/ml à 30 mg/ml de cyclosporine A et de 37,5 mg à 225 mg/ml d'un phospholipide, dans laquelle le diamètre aérodynamique moyen en masse dudit liposome constituant l'aérosol est de 1,0 µm à 3,0 µm.

2. Composition de liposomes à cyclosporine A en aérosol selon la revendication 1, comprenant une concentration de départ dans le réservoir de 5 mg/ml à 21,3 mg/ml de cyclosporine A et de 37,5 mg à 160 mg/ml d'un phospholipide.

3. Composition de liposomes à cyclosporine A en aérosol selon la revendication 2, dans laquelle le phospholipide est la dilauroylphosphatidylcholine.

4. Composition de liposomes à cyclosporine A en aérosol selon la revendication 1, dans laquelle le rapport de la cyclosporine A au phospholipide est de 1 sur 7,5.

5. Composition de liposomes à cyclosporine A en aérosol selon la revendication 1, dans laquelle le phospholipide est choisi dans le groupe constitué par la phosphatidylcholine de jaune d'oeuf, la phosphatidylcholine de soja hydrogénée, la dimyristoylphosphatidylcholine, la dilauroylphosphatidylcholine, la dioléoyldipalmitoylphosphatidylcholine et la dipalmitoylphosphatidylcholine.

6. Composition de liposomes à cyclosporine A en aérosol selon la revendication 1, pour son utilisation dans le traitement d'une maladie des poumons à médiation immunologique.

7. Composition de liposomes à cyclosporine A en aérosol selon la revendication 6, où ladite maladie des poumons à médiation immunologique est choisie dans le groupe constitué par un rejet de greffe allogénique, une bronchiolite oblitérante, une allergie, des hypersensibilités et un asthme.
